(19) Europäisches Patentamt
European Patent Office
Office européen des brevets

(11) Publication number: **0 341 393 B1**

(12) **EUROPEAN PATENT SPECIFICATION**

(45) Date of publication of patent specification: **17.03.93**

(51) Int. Cl.5: **C07C 43/04**, C07C 41/09, B01J 23/36

(21) Application number: **89104333.3**

(22) Date of filing: **11.03.89**

(54) **Method for the single step synthesis of methyl-tert.butyl ether from tert.butanol using supported rhenium catalysts.**

(30) Priority: **14.03.88 US 167948**

(43) Date of publication of application:
**15.11.89 Bulletin 89/46**

(45) Publication of the grant of the patent:
**17.03.93 Bulletin 93/11**

(84) Designated Contracting States:
**DE ES FR GB IT NL**

(56) References cited:
**GB-A- 403 402**
**US-A- 2 282 469**
**US-A- 4 144 138**
**US-A- 4 173 587**

(73) Proprietor: **TEXACO DEVELOPMENT CORPORATION**
**2000 Westchester Avenue**
**White Plains, New York 10650(US)**

(72) Inventor: **Knifton, John Frederick**
**10900 Catskill Trail**
**Austin Texas 78761(US)**
Inventor: **Sanderson, John Ronald**
**15290 Faubion Trail**
**Leander Texas 78641(US)**

(74) Representative: **Brock, Peter William et al**
**UROUHART-DYKES & LORD 91 Wimpole Street**
**London W1M 8AH (GB)**

**EP 0 341 393 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention concerns an improved process for preparing methyl tertiary butyl ether by the reaction of tertiary butanol and methanol in the presence of a catalyst comprising insoluble rhenium on an inert support. The invention is particularly advantageous in that the reaction takes place in one-step, the catalyst exhibits excellent selectivity to the desired ether product and high levels of tert-butanol conversion are achieved.

It is known to those skilled in the art that ethers, including unsymmetrical ethers, may be prepared by reacting an alcohol with another alcohol to form the desired product. The reaction mixture, containing catalyst and/or condensing agent may be separated and further treated to permit attainment of the desired product. Such further treatment commonly includes one or more distillation operations.

Methyl tert-butyl ether is finding increasing use as a blending component in high octane gasoline as the current gasoline additives based on lead and manganese are phased out. Currently all commercial processes for the manufacture of methyl tert-butyl ether (MTBE) are based upon the liquid-phase reaction of isobutylene and methanol (eq. 1), catalyzed by a cationic ion-exchange resin (see, for example: Hydrocarbon Processing, Oct. 1984, p. 63; Oil and Gas J., Jan. 1, 1979, p. 76; Chem. Economics Handbook-SRI, Sept. 1986, p. 543-705:P). The cationic ion-exchange resins used in MTBE synthesis normally have the sulphonic acid functionality (see: J. Tejero, J. Mol. Catal., 42 (1987) 257; C. Subramamam et al., Can. J. Chem. Eng., 65 (1987) 613).

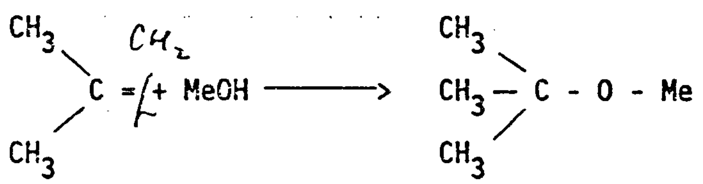

(Eq. 1)

With the expanding use of MTBE as an acceptable gasoline additive, however, a growing problem is the availability of raw materials. Historically, the critical raw material is isobutylene (Oil and Gas J., June 8, 1987, p. 55). It would be advantageous, therefore, to have a process to make MTBE that does not require isobutylene as a building block. Specifically, it would be advantageous to have an efficient process for making MTBE by reaction of methanol with tertiary butyl alcohol, since t-butanol is readily available commercially through isobutane oxidation.

In U. S. Patent No. 4,144,138 (1979) to Rao et al., there is disclosed a method for recovering methyl tertiary butyl ether from etherification reaction effluent by azeotropic distillation to recover methanol-ether azeotrope overhead which is water-washed to give pure ether raffinate, the latter being azeotropically distilled to yield ether-methanol overhead which is recycled to water washing.

The most closely related art appears to be the preparation of methyl tert-butyl ether from methyl and tert-butyl alcohols as discussed in S. V. Rozhkov et al., Prevrashch Uglevodorodov, Kislotno-Osnovn. Geterogennykh Katal. Tezisy Dokl. Vses Konf., 1977, 150 (C. A. 92:58165y). Here the TBA and methanol undergo etherification over KU-2 strongly acidic sulfopolystyrene cation-exchangers under mild conditions. This reference contains data on basic parameters of such a process. Problems with this method appear to include the following: 1) Athough a plant for etherification over cation exchangers does not present any problems, considerations include the fact that recycling large amounts of tert-butyl alcohol and methanol, as well as isobutylene, causes the scheme to be somewhat more expensive. 2) The progress of the reaction over cation exchangers is usually complicated by various adsorption and diffusion factors, by swelling phenomena, and by the variable distribution of the components between the solution and ion-exchanger phase. 3) Said acidic cation-exchangers with an organic (polystyrene or polymethacrylate) backbone generally have a very limited stability range with regard to operating temperatures, with temperatures above 120 °C normally leading to irreversible destruction of the resin and loss of catalytic activity.

In U. S. Patent No. 4,173,587 a method is described for cleaving cumene hydroperoxide to phenol and acetone using rhenium on a support group consisting of alumina, zirconia, silica-alumina or magnesia-alumina. In this patent, at Col. 2, lines 35-36, it is taught that a support containing carbon results in undesirably low yields. Therefore, it is not expected that rhenium-on-carbon would be a useful catalyst for other acid-catalyzed reaction, such as for MTBE production.

It would be a substantial advance in the art if methyl tertiary butyl ether could be selectively synthesized commercially in a continuous process from tertiary butyl alcohol and methanol in one step using an inorganic, heterogeneous, catalyst that was thermally stable to temperatures above 120 ° C, preferably to temperatures up to 200 ° C.

In accordance with certain of its aspects, the novel method of this invention for preparing methyl tert-butyl ether from tertiary butyl alcohol (t-butanol) and methanol in one-step comprises reacting tertiary butyl alcohol and methanol in the presence of a catalyst comprising an insoluble source of rhenium on carbon at an elevated temperature and moderate pressure.

Preparation of the product of this invention may be carried out typically by reacting tertiary butyl alcohol and methanol in the presence of an insoluble rhenium catalyst deposited on carbon. The etherification is carried out in one step.

The reaction can be represented by the following:

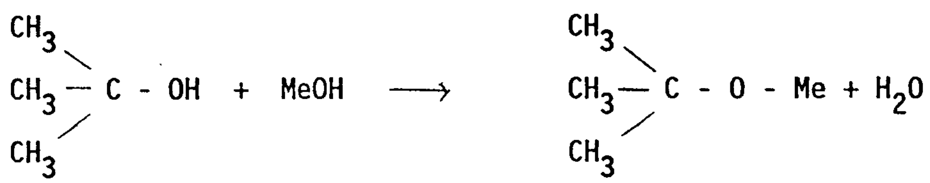

(Eq. 2)

The molar ratio of methanol to t-butanol in the feed mixture is between 10:1 and 1:10, so that the yield of desired MTBE is maximized. In order to achieve maximum selectivity to MTBE, and optimum conversion per pass, an excess of methanol in the liquid feed is desirable. The most preferred methanol-to-tertiary butanol molar ratio is from 1:1 to 5:1.

The rhenium used in the method of this invention should be in an insoluble form. If rhenium dissolves in the reaction product it could represent a significant economic loss. In addition, it is a substantial economic burden to recover, purify and reactivate a rhenium catalyst, because the element is costly.

Typically, the supported rhenium catalyst is prepared by inpregnating the rhenium onto a carbon support using a solution of rhenium compound, followed by drying, calcining and reduction.

Suitable rhenium compounds include rhenium oxides, salts, complexes and perrhenate compounds. They include rhenium heptoxide, ammonium perrhenate, rhenium(III) bromide, rhenium(III) chloride, rhenium(1V) chloride, decacarbonyldirhenium, rhenium(IV) oxide; rhenium(VI) oxide, rhenium(VII) selenide, rehnium(IV) sulfide, cyclopentadienylrhenium tricarbonyl, perrhenic acid and pentamethylcyclopentadienylrhenium tricarbonyl.

These rhenium compounds may be solubilized in aqueous, acid and organic solvents, prior to addition to the inert support.

The support is a carbon-containing support. Good results have been achieved with carbon as the support, as demonstrated in Example I. Said supports are preferably of high purity and high surface area (>10m$^2$/g).

The weight percent of rhenium to carbon support should be such that the concentration of the rhenium on the support is in the range of 0.1 wt% to 30 wt%, more preferably from 1-20 wt%.

In a typical catalyst preparation, a solution of ammonium perrhenate in water can be introduced onto carbon by impregnation. The concentration of the rhenium in the solution and the amount of the solution is adjusted to obtain a supported catalyst containing from 1% to 25% rhenium determined as the metal. However, it is preferred that the catalyst contain from 1 to 20% of the rhenium determined as the metal.

The rhenium may be impregnated in the manner described, however there are several supported rhenium catalysts commercially available. For example, a 10% rhenium-on-carbon catalyst is available from Strem Chemical Company.

Where the rhenium is impregnated on the support it has been found that the supported rhenium catalyst of our invention can in some cases be made more selective if the support is treated with an alkali metal or alkaline earth metal compound prior to the addition of rhenium to the support. Sodium and potassium are the preferred alkali metals and calcium, strontium and barium are the preferred alkaline earth metals with magnesium being less desirable because of a tendency to leach from the support. The support can be impregnated with an aqueous solution of the alkali metal or alkali metal compounds, such as the hydroxide, carbonate, bicarbonate and the like, then dried and calcined. The amount of the alkali metal or alkaline earth metal that is incorporated into the support is up to 5% metal based on the final catalyst, preferably 0.5 to 2.5% metal. Following this treatment, the rhenium can then be incorporated on the

support. However, the support can also be successfully treated with the alkali or alkaline earth metal concurrently with or subsequent to the treatment with the rhenium compound. The support can conveniently be dried after both the alkali or alkaline earth metal impregnating step and the rhenium impregnating step at a temperature between 100° and 200°C and calcined at a temperature between 200° and 450°C.

The reaction may be carried out in either a stirred slurry reactor or in a fixed bed continuous flow reactor. The catalyst concentration should be sufficient to provide the desired catalytic effect.

Etherification is conducted at temperatures from 20° to 200°C; the preferred range is 200° to 180°C. The total operating pressure is from 0 to 1000 psig, (100 to 6995 kPa). The preferred pressure range is 50 to 500 psig (445 to 3550 kPa).

Typically, MTBE is generated continuously in up to 25+ wt% concentration in the crude liquid product at total liquid hourly space velocities (LHSV) of up to four and relatively mild conditions, where:

$$LHSV = \frac{\text{Volume of Total Liquid Feed Run Through The Reactor Per Hour}}{\text{Volume of Catalyst In Reactor}}$$

The examples which follow illustrate the one-step synthesis of MTBE from TBA and MeOH (Eq. 2) using supported rhenium catalysts, particularly rhenium-on-carbon.

Conversion of t-butanol (TBA, wt%) is estimated in the following examples using the equation:

$$\frac{\text{(Wt\% Conc. of TBA in Feed - Wt\% Conc. of TBA in Product)}}{\text{Wt\% Conc of TBA in Feed}} \times 100$$

Yields of methyl t-butyl ether (MTBE, mole %) are estimated from:

$$\frac{\text{Moles of MTBE in Product Liquid}}{\text{moles of TBA converted}} \times 100$$

In samples in Example I, rhenium (10%)-on-carbon from Strem Chemicals, gave MTBE in ca. 25% concentration when run at LHSV of 1 (eq. Sample #12) and ca. 26% concentration in the crude liquid product when run at 180°C (eq. Sample #14). The operating conditions in both cases are quite moderate.

For Sample #12, analyses show:

Estimated TBA conversion per pass = 55%
MTBE yield (basis TBA converted) = 74 mole %.

For Sample #14, analyses show:
Estimated TBA conversion per pass = 85%
MTBE yield (basis TBA converted) = 50 mole %.

Syntheses were also conducted at 150°C, LHSV = 4.

A typical MTBE preparation procedure is given in Example I.

In the samples in Example 2, (for Comparison) rhenium (0.5%)-on-alumina from Strem Chemicals, exhibited essentially no activity during screening for MTBE production from TBA/MeOH (see Table I).

## EXAMPLE I

This example illustrates the cosyntheses of methyl t-butyl ether from t-butanol and methanol using a rhenium-on-carbon catalyst.

The synthesis was conducted in a tubular reactor (0.563" (14mm) id; 12" (305mm) long), constructed of 316 stainless steel, operated upflow and mounted in a furnace, controllable to ±1.0°C and fitted with pumps allowing flow control to <±1 $cm^3$/h. The reactor was also fitted with a pressure regulating device and equipment for monitoring temperature, pressure and flow rate.

The reactor was charged at the beginning of the experiment with 25 cc of rhenium (10%)-on-carbon catalyst (from Strem Chemicals). A screen of glass beads was placed at the top and bottom of the reactor to ensure the solid catalyst would remain in the middle portion.

The catalyst bed was first conditioned overnight by washing with methanol/t-butanol (2:1 mix) at 100 °C, 300 psi (2069 kPa) back pressure and a liquid flow rate of 25 $cm^3$/h. The same solution of methanol (1281.6g, 40.0 mole) plus t-butanol (1482.4g, 20.0 mole) was then pumped through the catalyst bed at 25 $cm^3$/h., while the reactor was held at 100 °C, at a total pressure of 300 psi (2069 kPa). Samples of product were taken periodically, either by trapping in a dry ice cooled container, or by collecting on-stream in a 316 ss bomb. Typical analyses data for samples taken under these conditions are summarized in Table 1. Catalyst performance at other operating temperatures and liquid flow rates were also measured, after reaching equilibrium conditions overnight. Summary data for these runs are also given in Table I.

EXAMPLE II (for Comparison)

The example illustrates the poor performance for MTBE synthesis of a rhenium-on-alumina catalyst.

Following the procedures of Example I, the same reactor is charged with 50 cc of rhenium(0.5%)-on-alumina catalysts (from Strem Chemical). The catalyst was first conditioned overnight by washing with methanol/t-butanol (2:1 mix) at 100 °C, 300 psi (2069 kPa) back pressure and a liquid flow of 25 $cm^3$/h. The same solution of methanol (40.0 mole) plus t-butanol (20 mole) was then pumped through the catalyst bed at 25 $cm^3$/h, while the reactor was held at 120 °c, at a total pressure of 300 psi (2069 kPa). Samples were taken periodically either by trapping or collecting on-stream. Typical data are summarized in Table I.

Catalyst Performance was also measured at 150° and 180° C.

TABLE I

| Example | Catalyst | Feed Sample | Flow Rate ($cm^3$/h) | Pressure (psig) (kPa) | Temp. (°C) | Sample | PRODUCT COMPOSITION (WT%) MTBE | $i\text{-}C_4$ | MeOH | tBA | $H_2O$ |
|---|---|---|---|---|---|---|---|---|---|---|---|
| | | F | | | | | | | 47.4 | 52.5 | |
| I | Re/C[a] | | 25 | 300 (2069) | 100 | #1 | 10.4 | 2.9 | 43.1 | 39.4 | 3.9 |
| | | | | | | #4 | 9.2 | 1.6 | 43.9 | 42.5 | 2.6 |
| | | | | | | #6 c | 7.3 | 1.2 | 44.9 | 44.8 | 1.7 |
| | | | " | " | 150 | #7 | 20.5 | 6.1 | 38.5 | 27.9 | 6.9 |
| | | | | | | #11 | 23.4 | 4.4 | 38.6 | 25.6 | 7.9 |
| | | | | | | #12 c | 25.1 | 5.8 | 37.4 | 23.8 | 7.9 |
| | | | " | " | 180 | #14 | 26.4 | 12.8 | 38.8 | 8.0 | 13.9 |
| | | | | | | #17 | 25.1 | 16.2 | 38.2 | 6.7 | 13.6 |
| | | | | | | #18 c | 23.5 | 15.3 | 39.3 | 7.7 | 14.1 |
| | | | 100 | " | 150 | #21 | 10.9 | 2.7 | 43.5 | 39.3 | 3.5 |
| | | | | | | #23 | 10.4 | 2.5 | 43.5 | 40.0 | 3.4 |
| | | | | | | #24 c | 10.8 | 2.8 | 43.5 | 39.7 | 3.2 |
| | | F | | | | | | | 46.2 | 53.7 | |
| II (Comparison). | $Re/AL_2O_3$[b] | | 25 | 300 (2069) | 120 | #2 | 1.1 | 0.2 | 45.7 | 52.0 | 0.9 |
| | | | | | | #7 | - | - | 46.2 | 53.7 | - |
| | | | | | | #9 c | - | - | 46.2 | 53.1 | - |
| | | | " | " | 150 | #10 | - | - | 46.3 | 53.6 | - |
| | | | | | | #13 | - | - | 46.0 | 53.7 | - |
| | | | | | | #15 c | - | - | 46.2 | 53.1 | - |
| | | | " | " | 180 | #16 | - | 0.1 | 46.2 | 53.4 | 0.1 |
| | | | | | | #19 | - | - | 45.9 | 53.6 | 0.4 |
| | | | | | | #21 c | 0.5 | - | 46.5 | 52.9 | - |

a A 10% Rhenium-On-Carbon Catalyst (Strem Chemical)
b A 0.5% Rhenium-On-Alumina Catalyst (Strem Chemical)
c On-Line Sample

## Claims

1. A method for producing methyl tertiary butyl ether continuously by reacting tertiary butyl alcohol and methanol in a molar ratio of 1:10 to 10:1 in the presence of a catalyst characterised in that the catalyst comprises an insoluble source of rhenium impregnated on carbon in an amount of from 0.1 to 30 wt% of the total weight of the catalyst,and in that reaction is carried out at a temperature of 20 to 200 °C and a pressure of 0 to 1000 psi (100 to 6995 kPa).

2. A method according to Claim 1 characterised in that the support has been treated before, during or after incorporating the rhenium, with a compound of an alkali metal or alkaline earth metal.

3. A method according to Claim 1 or 2 characterised in that the ratio of tertiary butanol to methanol is 1:1 to 1:5.

## Patentansprüche

1. Verfahren zur kontinuierlichen Herstellung von Methyltert.-butylether durch Umsetzen von tert.-Butylalkohol und Methanol in einem Molverhältnis von 1:10 zu 10:1 in Gegenwart eines Katalysators, dadurch gekennzeichnet, daß der Katalysator eine unlösliche Rheniumquelle umfaßt, die auf Kohlenstoff in einer Menge von 0,1 bis 30 Gew.-% des Gesamtgewichts des Katalysators imprägniert ist, und daß die Reaktion bei einer Temperatur von 20 bis 200 °C und einem Druck von 0 bis 1000 psi (100 bis 6995 kPa) durchgeführt wird.

2. Verfahren nach Anspruch 1, dadurch gekennzeichnet, daß der Träger vor, während oder nach Einbau des Rheniums mit einer Verbindung eines Alkalimetalls oder Erdalkalimetalls behandelt worden ist.

3. Verfahren nach Anspruch 1 oder 2, dadurch gekennzeichnet, daß das Verhältnis von tert.-Butanol zu Methanol 1:1 bis 1:5 ist.

## Revendications

1. Procédé de production de méthyltertiobutyléther en continu en faisant réagir l'alcool butylique tertiaire et le méthanol dans un rapport molaire de 1:10 à 10:1 en présence d'un catalyseur caractérisé en ce que le catalyseur comprend une source insoluble de rhénium imprégnée sur du carbone dans une quantité allant de 0,1 à 30% en poids du poids total de catalyseur et en ce que la réaction est effectuée à une température de 20 à 200 °C et à une pression de 0 à 1000 psi (100 à 6995 kPa).

2. Procédé selon la revendication 1 caractérisé en ce que le support a été traité avant, pendant ou après incorporation du rhénium, avec un composé d'un métal alcalin ou d'un métal alcalino-terreux.

3. Procédé selon la revendication 1 ou 2 caractérisé en ce que le rapport du butanol tertiaire au méthanol est de 1:1 à 1:5.